# EUROPEAN PATENT APPLICATION

(11) **EP 1 690 533 A1**
(43) Date of publication of application: **16.08.2006**
(21) Application number: 04793405.4
(22) Date of filing: 29.10.2004
(51) Int. Cl.: A61K 31/09, A61K 31/075, A61K 9/08, A61K 9/14, A61K 9/16, A61K 9/48, A23L 1/30, A23K 1/165, A23K 1/16, A61P 1/16

(54) **COMPOSITION HAVING LIVER FUNCTION PROTECTIVE EFFECT**

(30) Priority: 28.11.2003 JP 2003398485
(71) Applicant: KANEKA CORPORATION, Osaka-shi, Osaka 530-8288 (JP)
(72) Inventor: KUBO, Hiroshi, Tarumi-ku, Kobe-shi, Hyogo 655-0872 (JP); FUJII, Kenji, Kita-ku, Kobe-shi, Hyogo 651-1202 (JP); HOSOE, Kazunori, Takasago-shi, Hyogo 676-0025 (JP)
(74) Representative: HOFFMANN EITLE
(86) International application number: PCT/JP2004/016485
(87) International publication number: WO 2005/051370

(57) **Abstract**

The present invention provides a highly safe composition which is effective for protecting liver functions and preventing liver dysfunctions in the case where liver dysfunctions rise by various causes. The present inventors found that oxidized coenzyme Q is effective for protecting liver functions in the absence of selenium and that reduced coenzyme Q has a higher efficacy for protecting liver functions. The composition containing, as the main components, oxidized coenzyme Q and less than 0.01 percent by weight of selenium, or reduced coenzyme Q can be used to protect liver functions. The present invention can provide a highly safe composition which is highly effective for maintaining health.

## Description

### TECHNICAL FIELD

The present invention relates to compositions which contain coenzyme Q and which are effective for protecting liver functions.

### BACKGROUND ART

Liver is an important organ responsible for various biochemical activities essential for living body, and therefore likened to "a chemical factory in the living body". The liver produces bile and regulates the metabolism and storage of three major nutrients, carbohydrate, protein, and fat. It decomposes metabolic waste products, detoxifies noxious substances, and controls hematopoiesis and the flow of blood. Damage on the liver caused by viral infection, excessive intake of alcohol, drug intoxication, irregular eating habits, stress, smoking, or the like, leads to liver dysfunction, which eventually develops into a liver disease such as acute hepatitis, chronic hepatitis, alcohol-induced fatty liver, or liver cancer.

Once liver cells are damaged by a virus, alcohol, drug, or the like, an enzyme, such as glutamic-oxaloacetic transaminase (GOT) or glutamic-pyruvic transaminase (GPT), in the cells flows into blood, thereby increasing enzymatic activity in blood. GOT or GPT activity in blood has thus been widely used as an indicator of liver dysfunction.

As the drugs effective for prevention or cure of the liver dysfunction, glutathione, various antiviral agents, and various immunosuppressive agents etc. have been known. Turmeric, sesamin, lady's-thistle, and sesame lignan etc. have also been known to protect, enhance, or improve the liver function.

Coenzyme Q is an essential ingredient found in a wide diversity of living organisms from bacteria to mammals and is known to function as a constituent of the electron transfer system of mitochondria in the cells of living organisms. Coenzyme Q not only functions as a transfer agent in the electron transfer system by repeating oxidation and reduction in the mitochondria but also has an antioxidative effect when coenzyme Q is in the reduced form. Coenzyme Q₁₀ found mainly in human has a side chain consisting of ten repeating units.

Coenzyme Q₁₀ is characterized by its high safety. A chronic toxicity test conducted at a daily dosage of 1,200 mg/kg continuously for 52 weeks in rats reported no toxic effects (K. D. Williams et al., J. Agric. Food Chem., 47, 3756-3763, 1999). The dosage of 1,200 mg/kg in a rat is equivalent to a dosage of 60 g/day in a human subject weighing 50 kg. Considering the fact that the dosage of coenzyme Q₁₀ in Europe and the U.S. is 100 to 300 mg/day, there is no ambiguity about the safety of coenzyme Q₁₀.

The oxidized form of coenzyme Q₁₀ is widely used as a drug for treating congestive heart failure in Japan and as a health food in Europe and the U.S. Regarding the use of coenzyme Q₁₀ in the protection of liver functions, the following reports are available: (1) Liver dysfunction induced by carbon tetrachloride was suppressed by intraperitoneal administration of oxidized coenzyme Q₁₀ in rats (T. Takahashi et al., Biol. Pharm. Bull., 19 1005-1012, 1996); and (2) A drug containing oxidized coenzyme Q₁₀ and 0.01 to 0.1 percent by weight of selenium was administered to patients with liver diseases (Japanese Unexamined Patent Application Publication No. 11-199477); etc. However, there has been no study on the efficacy of coenzyme Q₁₀ to liver dysfunction when a composition containing oxidized coenzyme Q₁₀ and substantially no selenium is orally administered, or on the efficacy of the reduced form of coenzyme Q₁₀ in protecting liver functions.

The reduced form of coenzyme Q₁₀ is the resulting product from the reduction of oxidized coenzyme Q₁₀. Because reduced coenzyme Q₁₀ is readily converted into oxidized coenzyme Q₁₀ in air by oxidation, it has not been used in products. We have previously found that the overall absorbability of coenzyme Q₁₀ can be increased by mixing reduced coenzyme Q₁₀ with oxidized coenzyme Q₁₀ compared to when oxidized coenzyme Q₁₀ is used alone (Japanese Unexamined Patent Application Publication No. 10-109933). However, whether reduced coenzyme Q₁₀ exhibits a higher efficacy for protection of liver functions than oxidized coenzyme Q₁₀ remains unknown.

### SUMMARY OF THE INVENTION

An object of the present invention is to provide a highly safe substance effective for protecting liver functions.

To achieve the object, the present inventors have conducted extensive investigations and found that oxidized coenzyme Q is effective for protecting liver functions in the absence of selenium and that reduced coenzyme Q has a higher efficacy for protecting liver functions.

That is, the present invention relates to a composition for protecting liver functions, the composition containing reduced coenzyme Q represented by formula (1): 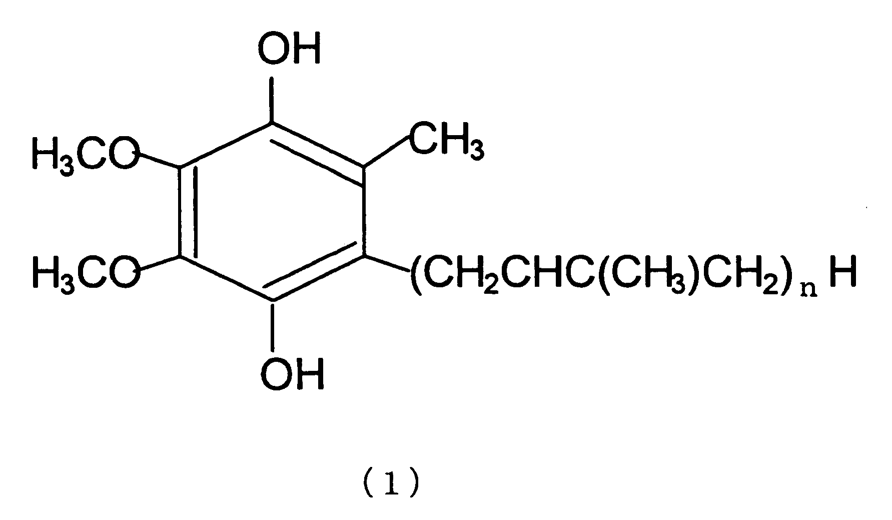 (wherein n represents an integer in the range of 1 to 12).

One aspect of the present invention provides an agent for protecting liver functions, the agent containing the above-mentioned reduced coenzyme Q as an active ingredient.

Furthermore, the present invention provides a composition for protecting liver functions, the composition containing oxidized coenzyme Q represented by formula (2) 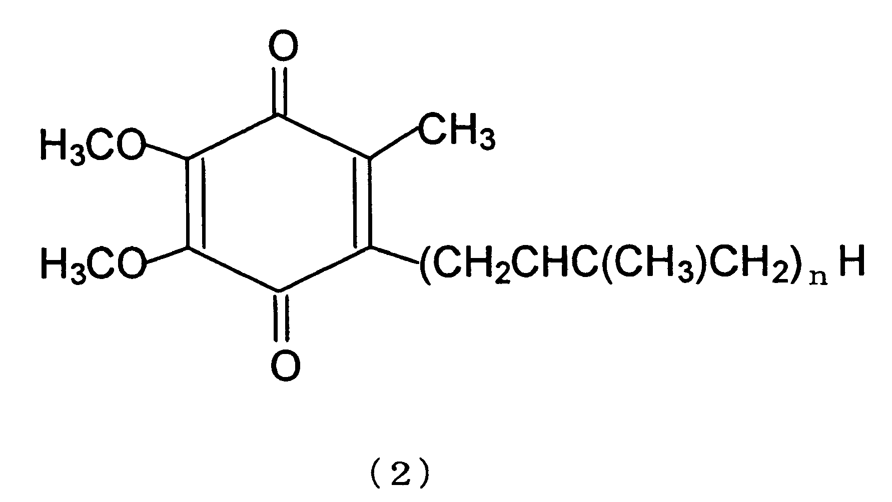 and selenium, wherein the content of the selenium is less than 0.01 percent by weight.
(wherein n represents an integer in the range of 1 to 12).

One aspect of the present invention provides an agent for protecting liver functions, the agent containing oxidized coenzyme Q as an active ingredient.

### DETAILED DESCRIPTION OF THE INVENTION

In the present invention, "protecting liver functions" includes protecting liver functions from disorders and preventing liver dysfunctions. An example of the former is to reduce an increase in GPT or GOT activity in blood induced by carbon tetrachloride administration.

Coenzyme Q is represented by either one of the following formulae: 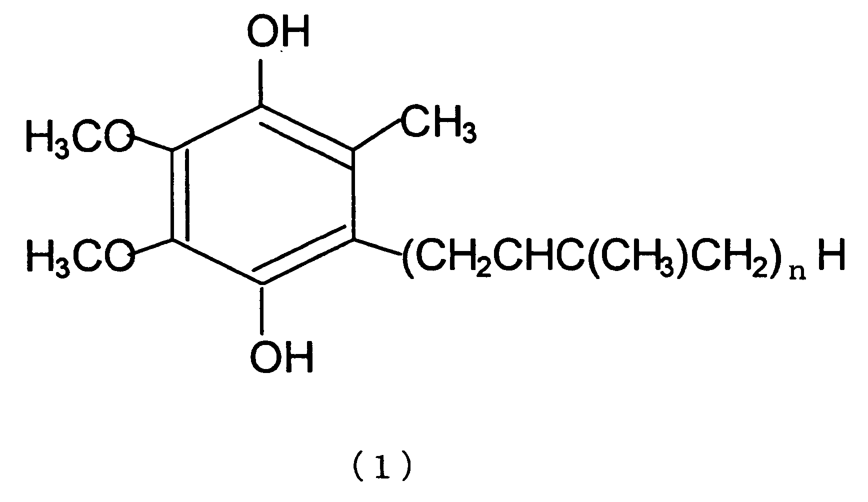 (wherein n represents an integer of 1 to 12) and 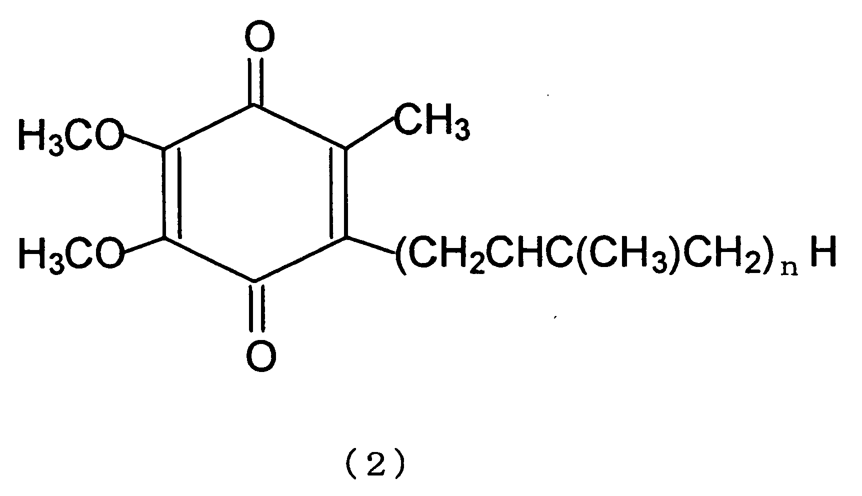 (wherein n is as defined above). Formula (1) represents the reduced form of coenzyme Q and formula (2) represents the oxidized form of coenzyme Q. A nonlimiting example of the process for obtaining oxidized coenzyme Q or reduced coenzyme Q includes the steps of obtaining coenzyme Q by a conventional process, such as synthesis, fermentation, or extraction from natural materials, and then concentrating oxidized or reduced coenzyme Q fraction in the effluent by chromatography. Oxidized coenzyme Q may be prepared by any other known methods (Japanese Unexamined Patent Application Pubication No. 53-38690). In order to prepare reduced coenzyme Q, a common reductant, such as sodium borohydride or sodium dithionite (sodium hydrosulfite) may be added to oxidized coenzyme Q according to need in order to reduce the oxidized coenzyme Q for obtaining reduced coenzyme Q by conventional methods, followed by chromatographic concentration. It is also possible to obtain reduced coenzyme Q by the reaction between a high-purity oxidized coenzyme Q product and the above-described reductant.

Coenzyme Q usable in the present invention has, as shown in formulae (1) and (2), a side chain containing 1 to 12 repeating units (the number of which is indicated as n in these formulae). Coenzyme Q having ten repeating units in the side chain, i.e., coenzyme Q₁₀, is particularly preferred. This is because coenzyme Q having ten repeating units in the side chain, i.e., coenzyme Q₁₀, is mainly contained in human.

The composition for protecting liver functions according to the present invention (hereinafter referred to as the "inventive composition") may contain reduced coenzyme Q. Alternatively, the inventive composition may contain oxidized coenzyme Q and less than 0.01 percent by weight of selenium.

In the inventive composition containing reduced coenzyme Q, the content of reduced coenzyme Q is preferably 0.001 to 99 percent by weight and more preferably 0.01 to 20 percent by weight.

In the inventive composition containing oxidized coenzyme Q and less than 0.01 percent by weight of selenium, the content of oxidized coenzyme Q is preferably 0.001 to 99 percent by weight and more preferably 0.01 to 20 percent by weight.

The inventive composition may contain reduced coenzyme Q and oxidized coenzyme Q. The content of reduced coenzyme Q to the total of coenzyme Q is not particularly limited but is preferably at least 50 percent by weight, more preferably at least 60 percent by weight, yet more preferably at least 70 percent by weight, and most preferably at least 80 percent by weight.

The inventive composition containing oxidized coenzyme Q and no selenium is effective for protecting liver functions. The selenium content in the composition is preferably less than 0.01 percent by weight, and more preferably less than 0.001 percent by weight.

The composition with low or no selenium content is advantageous in the viewpoint that the undesirable affect on the living body by selenium is low.

Selenium content in compositions can be measured by the following method. Appropriate doses of samples are digested with 10 ml concentrated nitric acid in digesting tubes, and left at ambient temperature overnight. By gradually increasing the temperature of the tube to 130°C, a dense brown fume appeared. The temperature is maintained until the volume of the solution is reduced to 1 ml. The tubes are cooled to room temperature and 3 ml of perchloric acid is added. They are then gradually heated to 150°C. The temperature is maintained until the brown fume NO₂ is replaced by the white fume of perchloric acid and until the sample volume is reduced to 1-2 ml. The tubes are cooled to room temperature and 2 ml of 6 N hydrochloric acid is added. They are reheated to 130°C for 1 h. The pH of the digested samples is adjusted to 1-2 with 6 N ammonia solution. Selenium is complexed with 0.1% diaminonapthalene (DAN) solution at 50°C for 20 min and extracted with cyclohexane. Cyclohexane phase containing Se-DAN complex is then measured spectrofluorometrically at an excitation wavelength 380 nm and emission wavelength 520 nm and selenium content in samples is determined.

Contents of reduced coenzyme Q₁₀ and oxidized coenzyme Q₁₀ in compositions can be measured by the following method. To 50 mg of sample, 0.1 ml of water, 0.4 ml of methanol and 0.6 ml of n-hexane were added in the order mentioned. The mixture was shaken vigorously for about 30 seconds and then centrifuged to separate into two layers. A certain volume of the organic layer was taken and immediately evaporated to dryness using nitrogen gas. To the residue was added 0.2 ml of ethanol for use as an assay sample. The assay of reduced coenzyme Q₁₀ and oxidized coenzyme Q₁₀ was carried out by high performance liquid chromatography under the following conditions.
Column: 250 mm long, 4.6 mm in diameter YMC-Pack ODS-A (product of YMC Co., Ltd.)
Mobile Phase: Methanol:Hexane(85:15,v:v)
Detection wavelength: 290 nm
Flow rate: 1.0ml/min.

The inventive composition can be used as, for example, a pharmaceutical agent, a functional food, a food material, or an animal feedstuff. Here, the "functional foods" include any nonpharmaceutical substances that can be taken orally to maintain or improve health. Examples of the functional foods include oral supplements, foods for specified health use, health foods, and dietary supplements.

The content of coenzyme Q in the inventive composition, and the dosage form, the preservation method, and the preservation form of the inventive composition may be appropriately selected based on its use, for example, as a pharmaceutical agent, a health food, a food in general, an animal drug, or an animal feedstuff.

The dosage form of the pharmaceutical agent containing the inventive composition is not particularly limited. For example, the pharmaceutical agent may be liquid, powder, granules prepared using binders, coated powder prepared by coating powder with coating agents, or capsules containing powder, granules, or coated powder. Examples of the agent in the liquid form include, but are not limited to, health drinks, injectable solutions, and instillation solutions. The inventive composition may be blended with natural oil, an oily higher fatty acid, a higher fatty acid monoglyceride, a surfactant, or a mixture of these and the resulting oily mixture may be formed into a soft capsule. Here, a material mainly composed of gelatin or another water-soluble polymer may also be used. Moreover, the term "capsule" includes any microcapsule.

The inventive composition or the pharmaceutical agent may be administered in the form of liquid or solid by various methods, such as through mouth, injection, instillation, or suppository insertion. Alternatively, the inventive composition may be taken as part of food. Oral administration is generally preferred for its simplicity but, for example, where oral administration is not feasible, any other method such as injection may be employed without any disadvantage.

The inventive composition may be incorporated into a conventional liver-function-protecting food so that coenzyme Q is contained in the food. Among the inventive composition, a composition for protecting liver functions containing reduced coenzyme Q preferably contains an antioxidant to prevent oxidation of reduced coenzyme Q. Depending on the form of dosage, the antioxidant may not be necessary. Examples of the antioxidant usable include citric acid and derivatives thereof, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, superoxide dismutase (SOD), glutathione peroxidase, glutathione-S-transferase, glutathione reductase, catalase, ascorbate peroxidase, and any combination of these.

Among the inventive composition, a composition for protecting liver functions containing reduced coenzyme Q may contain a chelating agent that also prevents oxidation. Examples of the chelating agent usable include ethylenediaminetetraacetic acid and salts thereof, ethylenediaminediacetic acid and salts thereof, hydroxyiminodiacetic acid and salts thereof, hydroxyethylethylenediaminetetraacetic acid and salts thereof, diethylenetriaminepentaacetic acid and salts thereof, nitrilotriacetic acid and salts thereof, triethylenetetraaminehexaacetic acid and salts thereof, dicarboxymethyl glutamic acid tetrasodium salt, dihydroxymethylglycine, 1,3-propanediaminetetraacetic acid and salts thereof, 1,3-diamino-2-hydroxypropanetetraacetic acid and salts thereof, gluconic acid sodium salt, hydroxyethylidenediphosphonic acid, nitrilotris, phosphonobutanetricarboxylic acid, and mixtures thereof. The chelating agent and the antioxidant may be used in combination.

Adequate amounts of pharmaceutically acceptable and food-hygiene-acceptable materials, other than the above-mentioned coenzyme Q, may be added to and mixed with the inventive composition by a conventional process. Examples of such materials include, but are not limited to, excipients, disintegrants, lubricants, binders, coating agents, coloring agents, coagulation inhibitors, absorption promoters, solubilizing agents, stabilizing agents, health food materials, dietary supplement materials, and vitamins.

Examples of the excipient include, but are not limited to, sucrose, lactose, glucose, cornstarch, mannitol, crystalline cellulose, calcium phosphate, and calcium sulfate.

Examples of the disintegrants include, but are not limited to, starch, agar, calcium citrate, calcium carbonate, sodium hydrogen carbonate, dextrin, crystalline cellulose, carboxymethyl cellulose, and tragacanth.

Examples of the lubricants include, but are not limited to, talc, magnesium stearate, polyethylene glycol, silica, and hydrogenated vegetable oil.

Examples of the binders include, but are not limited to, ethyl cellulose, methyl cellulose, hydroxypropylmethyl cellulose, tragacanth, shellac, gelatin, gum arabic, polyvinyl pyrrolidone, polyvinyl alcohol, polyacrylic acid, polymethacrylic acid, and sorbitol.

Examples of the coating agents include, but are not limited to, gum arabic, opadry, prunella, castor wax, carboxyvinyl polymer, carmellose, hydrous silicon dioxide, magnesium silicate, vinyl acetate resin, stearic acid, cetanol, and hydroxypropylmethyl cellulose.

Examples of the coloring agents include, but are not limited to, those which are permitted to be added to drugs and foods.

Examples of the coagulation inhibitors include, but are not limited to, stearic acid, talc, light silicic anhydride, and hydrous silicon dioxide.

Examples of the absorption promoters include, but are not limited to, surfactants such as higher alcohols, higher fatty acids, and glycerin fatty acid esters.

Examples of the solubilizing agents include, but are not limited to, organic acids such as fumaric acid, succinic acid, and malic acid.

Examples of the stabilizing agents include, but are not limited to, benzoic acid, sodium benzoate, and ethyl parahydroxybenzoate.

Examples of health food materials include, but are not limited to, foods having protecting liver functions (e.g., flavangenol, Turmeric, sesamin, lady's-thistle, and sesame lignan), Chinese herb medicines (e.g., irei-to, unkei-to, unsei-in, ogi-kenchu-to, oren-gedoku-to, oren-to, ka-kkon-to, kami-kihi-to, kami-syoyo-san, kam-baku-taiso-to, kikyou-to, kihi-to, kumi-binro-to, keigai-rengyo-to, keihi-ka-syakuyakudaiou-to, keihi-ka-syakuyaku-to, keihi-ka-ryukotsu-borei-to, keishi-to, keishi-ninjin-to, keishi-bukuryo-gan, keihi-to, koso-san, goko-to, goshaku-san, gosha-jinki-gan, gorin-san, saikan-to, saiko-ka-ryukotsu-borei-to, saiko-keihi-kankyo-to, saiko-keishi-to, saiko-seikan-to, saiboku-to, sairei-to, sansonin-to, jiin-koka-to, shigyaku-san, shikunshi-to, shimotsu-to, sha-kanzo-to, shakuyaku-kanzo-to, juzen-daiho-to, jumi-haidoku-to, sho-kenchu-to, sho-saiko-to, sho-seiryu-to, shofu-san, shin'i-seihai-to, shimpi-to, shimbu-to, seijo-bufu-to, seisho-ekki-to, seishin-renshi-in, seihai-to, sokei-kakketsu-to, daio-kanzo-to, daio-botampi-to, dai-kenchu-to, dai-saiko-to, dai-saiko-to-kyo-daiou, dai-jouki-to, dai-bofu-to, ji-daboku-ippo, choi-joki-to, choto-san, choyo-to, chorei-to, chorei-to-go-shimotsu-to, tsu-do-san, tokaku-joki-to, toki-inshi, toki-kenchu-to, toki-shakuyaku-san, toki-to, nichin-to, nyoshin-san, ninjin-to, ninjin-yoei-to, haino-san-kyu-to, bakumondo-to, hachimi-jio-gan, hange-koboku-to, hange-shashin-to, byakko-ka-ninjin-to, bukuryo-in, bukuryo-in-go-hange-koboku-to, heii-san, boi-ogi-to, bofu-tsusho-san, hochu-ekki-to, mao-to, mao-bushi-saishin-to, makyo-kan-seki-to, mashinin-gan, moku-boi-to, yoku-kan-san, yokukan-san-ka-chimpi-hange, rikkushi-to, rikko-san, ryutan-shakan-to, ryo-kan-kyo-mi-shin-ge-nin-to, and rokumi-gan); teas (e.g., green tea, brown rice tea, powdered green tea, natural leaf tea, roasted tea, roasted tea, jasmine tea, oolong tea, red tea, black tea, floral tea, blue tea, and white tea); herbs (e.g., Italian parsley, elecampane, olive, oregano, cardoon, chamomiles, curry plant, catnip, caraway, christmas rose, crimson clover, cornflower, common mallow, salad burnet, santolina, cinnamon, jasmine, stevia, sage, linden, scented geranium, St. John's wort, soapwort, Solomon's seal, thyme, tansy, chervil, chive, nasturtium, jujube, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, lady's bedstraw, lemongrass, lemon verbena, lemon balm, rose, rosemary, rocket, wild strawberry, wild pansy, and forgetmenot); propolis; gingko leaves; green juice; and extracts thereof. Among these health food materials, preferred are foods effective for protecting liver functions (e.g., flavangenol, Turmeric, sesamin, lady's-thistle, and sesame lignan).

Examples of the dietary supplement materials include, but are not limited to, amino acids, metal ions, proteins, saccharides, fatty acids, yeast extracts, vegetable extracts, fish extracts, fruits, and fruit extracts.

Examples of the vitamins include, but are not limited to, vitamins A, D, E, K, B₁, B₂, B₆, B₁₂, and C, niacin, folic acid, biotin, pantothenic acid, and pyrrolo-quinoline quinone.

The liver function of the mammals such as human and other animals can be effectively protected by adequately using the composition of the present invention.

### BRIEF DESCRIPTION OF THE DRAWINGS

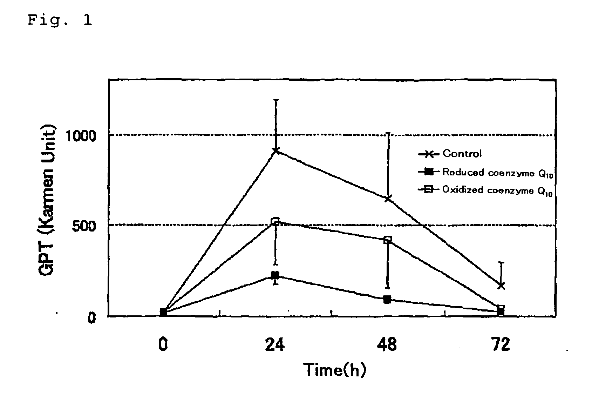

Fig. 1 is a graph showing the GPT activities in rat plasma samples plotted against time.

In the graph, the ordinate indicates GPT activities in the plasma samples, and abscissa indicates the time elapsed from the administration of carbon tetrachloride. The GPT activity profiles of three groups, namely, a solvent control group, a reduced coenzyme Q₁₀ (with about 2% of oxidized coenzyme Q₁₀) group, and an oxidized coenzyme Q₁₀ group, are shown with an average ± standard error (n = 4).

### BEST MODE FOR CARRYING OUT THE INVENTION

The present invention will now be described in further detail by EXAMPLE and PREPARATION EXAMPLES. These examples in no way limit the scope of the present invention.

### EXAMPLE 1

The effects of coenzyme Q₁₀ on protecting liver functions from disorders and preventing liver dysfunctions were examined by establishing a liver dysfunction model using carbon tetrachloride. A soy oil solution of oxidized coenzyme Q₁₀ or reduced coenzyme Q₁₀ with approximately 2% of oxidized coenzyme Q₁₀ was orally administered to male SD rats (6 weeks old) at a dosage of 100 mg/kg. About 16 hours after the administration, blood was collected from the jugular vein using heparin as an anticoagulant. After one hour, 50% carbon tetrachloride (product of Nacalai Tesque, Inc.)/olive oil (product of Wako Pure Chemical Industries, Ltd.) was intraperitoneally administered at a dosage of 2.0 ml/kg. Blood was collected 24 hours, 48 hours, and 72 hours after the administration of carbon tetrachloride. The collected blood samples were centrifuged to obtain plasma samples. The GPT activity of each plasma sample was measured using a kit, Transaminase CII-Test Wako (product of Wako Pure Chemical Industries, Ltd.).

Fig. 1 shows the GPT activities observed in the rat plasma samples.

In the oxidized coenzyme Q₁₀ group, the GPT activity in the plasma sample collected 24 hours after the administration of carbon tetrachloride was suppressed to about 60% of the control group. Similarly, in the reduced coenzyme Q₁₀ group, the GPT activity was suppressed to about 25%. The GPT activity in the plasma was the highest in the control group, the next highest in the oxidized coenzyme Q₁₀ group, and the lowest in the reduced coenzyme Q₁₀ group. This tendency was observed also at 48 hours and 72 hours after the administration. These results show that the liver dysfunction can be alleviated by administration of coenzyme Q₁₀. Moreover, reduced coenzyme Q₁₀ has a greater effect of alleviating liver dysfunction than oxidized coenzyme Q₁₀.

### PREPARATION EXAMPLE 1

Olive oil heated to 60°C was mixed with coenzyme Q₁₀ (reduced coenzyme Q₁₀:oxidized coenzyme Q₁₀ = 85:15) melted at 60°C. Vitamin E was gradually added to prepare a homogeneous mixture, and the homogeneous mixture was processed into soft capsules by a standard method. One soft capsule contained 20 mg of coenzyme Q₁₀, in particular,

| | |
|---|---|
| Coenzyme Q₁₀: | 20 parts by weight |
| Vitamin E: | 15 parts by weight |
| Olive oil: | 350 parts by weight |

### PREPARATION EXAMPLE 2

Coenzyme Q₁₀ (reduced coenzyme Q₁₀:oxidized coenzyme Q₁₀ = 85:15) was dissolved in acetone, adsorbed on crystalline cellulose (fine powder), and dried. The resulting product was mixed with cornstarch, and processed into powder by a standard method.

| | |
|---|---|
| Coenzyme Q₁₀: | 10 parts by weight |
| Crystalline cellulose: | 40 parts by weight |
| Cornstarch: | 55 parts by weight |

### PREPARATION EXAMPLE 3

Coenzyme Q₁₀ (reduced coenzyme Q₁₀:oxidized coenzyme Q₁₀ = 85:15) was dissolved in acetone, adsorbed on crystalline cellulose (fine powder), and dried. The resulting product was mixed with cornstarch, lactose, carboxymethyl cellulose, and magnesium stearate. An aqueous solution of polyvinyl pyrrolidone was added thereto to function as a binder, and the resulting mixture was processed into granules by a standard method. The granules were mixed with talc functioning as a lubricant. The resulting mixture was processed into tablets each containing 20 mg of coenzyme Q₁₀, in particular:

| | |
|---|---|
| Coenzyme Q₁₀: | 20 parts by weight |
| Cornstarch: | 25 parts by weight |
| Lactose: | 15 parts by weight |
| Carboxymethyl cellulose calcium: | 10 parts by weight |
| Crystalline cellulose: | 40 parts by weight |
| Polyvinyl pyrrolidone: | 5 parts by weight |
| Magnesium stearate: | 3 parts by weight |
| Talc: | 10 parts by weight |

### PREPARATION EXAMPLE 4

The materials below were processed into granules by a standard method and the resulting granules were packed in gelatin hard capsules, each containing 20 mg of coenzyme Q₁₀:

| | |
|---|---|
| Coenzyme Q₁₀ (reduced coenzyme Q₁₀:oxidized coenzyme Q₁₀ = 85:15): | 20 parts by weight |
| Crystalline cellulose: | 40 parts by weight |
| Cornstarch: | 20 parts by weight |
| Lactose: | 62 parts by weight |
| Magnesium stearate: | 2 parts by weight |
| Polyvinyl pyrrolidone: | 3 parts by weight |

### PREPARATION EXAMPLE 5

Olive oil heated to 60°C was mixed with oxidized coenzyme Q₁₀ melted at 60°C to prepare a mixture. Vitamin E was gradually added to the mixture to prepare a homogeneous mixture. The homogeneous mixture was processed into soft capsules by a standard method. One soft capsule contained 20 mg of oxidized coenzyme Q₁₀, in particular:

| | |
|---|---|
| Oxidized coenzyme Q₁₀: | 20 parts by weight |
| Vitamin E: | 15 parts by weight |
| Olive Oil: | 350 parts by weight |

### INDUSTRIAL APPLICABILITY

The present invention can provide a highly safe composition which is able to prevent and alleviate liver dysfunctions.

## Claims

1. A composition for protecting liver functions, which comprises reduced coenzyme Q represented by formula (1): 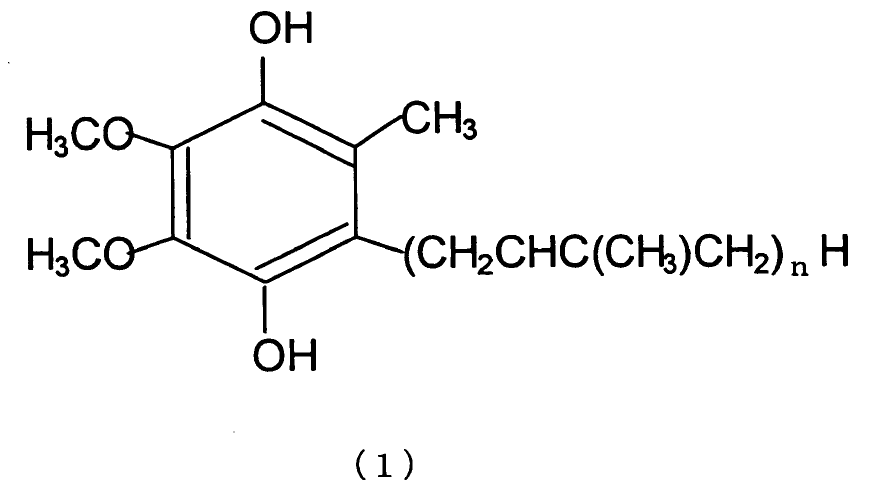 (wherein n represents an integer in the range of 1 to 12).

2. The composition according to claim 1, wherein the reduced coenzyme Q is reduced coenzyme Q₁₀.

3. The composition according to claim 1 or 2, wherein the content of the reduced coenzyme Q is in the range of 0.001 to 99 percent by weight.

4. The composition according to any one of claims 1 to 3, which further comprises a health food material.

5. A pharmaceutical formulation for protecting liver functions, which comprises the composition according to any one of claims 1 to 4.

6. The pharmaceutical formulation according to claim 5, which is in the form of a powder, or a capsule formulation.

7. The pharmaceutical formulation according to claim 5, which is in the form of a drinkable preparation, an injectable solution, or an infusion or a drops formulation.

8. A functional food for protecting liver functions, which comprises the composition according to any one of claims 1 to 4.

9. An animal feeding stuff for protecting liver functions, which comprises the composition according to any one of claims 1 to 4.

10. A method for protecting liver functions of a mammal, which comprises administering to a mammal the composition according to any one of claims 1 to 4 .

11. A method for protecting liver functions of a mammal, which comprises orally administering the composition according to any one of claims 1 to 4.

12. A composition for protecting liver functions, which comprises oxidized coenzyme Q represented by formula (2) and selenium, wherein the content of the selenium is less than 0.01 percent by weight: 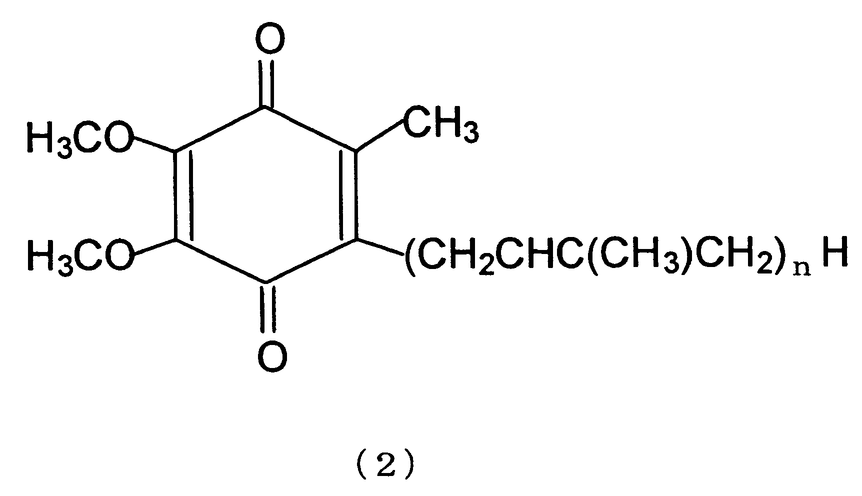 (wherein n represents an integer in the range of 1 to 12).

13. The composition according to claim 12, which is used for oral administration.

14. The composition according to claim 12 or 13, wherein the oxidized coenzyme Q is oxidized coenzyme Q₁₀.

15. The composition according to any one of claims 12 to 14, wherein the content of the oxidized coenzyme Q is in the range of 0.001 to 99 percent by weight.

16. The composition according to any one of claims 12 to 15, which further comprises a health food material.

17. A pharmaceutical formulation for protecting liver functions, which comprises the composition according to any one of claims 12 to 16.

18. The pharmaceutical formulation according to claim 17, which is in the form of a granulation, a powder, or a capsule formulation.

19. The pharmaceutical formulation according to claim 17, which is in the form of a drinkable preparation, an injectable solution, or an infusion or a drops formulation.

20. A functional food for protecting liver functions, which comprises the composition according to any one of claims 12 to 16.

21. An animal feeding stuff for protecting liver functions, which comprises the composition according to any one of claims 12 to 16.

22. A method for protecting liver functions of a mammal, which comprises administering to a mammal the composition according to any one of claims 12 to 16.
